(19) Europäisches Patentamt
European Patent Office
Office européen des brevets

(11) Publication number: **0 123 441 B1**

(12) **EUROPEAN PATENT SPECIFICATION**

(45) Date of publication of patent specification: **03.10.90**

(51) Int. Cl.$^5$: **C 07 D 519/04,** A 61 K 31/395, A 61 K 39/395

(21) Application number: **84302003.3**

(22) Date of filing: **26.03.84**

(54) **Vincaleukoblastine derivatives.**

(30) Priority: **30.03.83 GB 8308856**

(43) Date of publication of application: **31.10.84 Bulletin 84/44**

(45) Publication of the grant of the patent: **03.10.90 Bulletin 90/40**

(84) Designated Contracting States: **AT BE CH DE FR GB IT LI LU NL SE**

(56) References cited:
**EP-A-0 005 620**
**EP-A-0 041 935**
**US-A-3 387 001**

(73) Proprietor: **LILLY INDUSTRIES LIMITED**
**Erl Wood Manor**
**Windlesham Surrey GU20 6PH (GB)**

(72) Inventor: **Cullinan, George Joseph**
**R.R.1. Box 124B**
**Trafalgar Indiana 46181 (US)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

This invention relates to bifunctional ester derivatives of 4-desacetyl indole-dihydroindole alkaloids.

The alkaloids obtainable from *Vinca rosea* represent one of the most productive areas of chemistry for drugs which adversely affect the growth of experimental malignancies in mammals. Initially, only some of the alkaloids, obtainable from the leaves of the plant by extraction and purifiable by chromatography, were found to be active. These active antineoplastic alkaloids obtained directly from the leaves of the vinca plants include VLB (vinblastine, vincaleucoblastine), vincristine (leurocristine), leurosine (vinyleurosine), leurosidine (vinrosidine), leuroformine (formylleurosine) and deoxy VLB "A" and "B" (4'-deoxy VLB and 4'-deoxyleurosidine).

Chemical modification of the *Vinca* alkaloids started slowly for several reasons. In the first place, the molecular structures involved are extremely complex, and chemists were slow to find reactions which modified one specific functional group of the molecule without affecting other groups. Secondly, dimeric alkaloids lacking desirable chemotherapeutic properties had been recovered or produced from *Vinca rosea* extracts, and a determination of their structures had led to the conclusion that these inactive compounds were closely related structurally to and even isomeric with one or more of the active alkaloids. Thus, it appeared that small chemical changes in the known anticancer alkaloids could have a profound effect on antineoplastic activity.

Because of these restrictions, modification of the indole-dihydroindole alkaloids obtained from *Vinca rosea* has centered around only three positions on the molecule: C—3, C—4' and C—4. Considering C—3 modification first, one of the more recent, and more successful, modifications of the basic indoledihydroindole structure has been the preparation of C—3 carboxamide derivatives, most of which turned out to be active anti-tumor agents. [See US Patent 4,166,810, and Conrad et al. *J. Med. Chem. 22*, 391 (1979)]. 4-Desacetyl VLB 3-carboxamide (vinyldesine) is currently being marketed in several European countries as an oncolytic agent. It is said to be effective in treating some vincristine-resistant leukemias in addition to many common neoplasms including germ-cell tumors. Reaction of the 3-hydroxy and 3-ester functions with an isocyanate has produced the corresponding oxazolidinedione derivatives, one of which, the N-chloroethyl derivative (vinzolidine) is currently undergoing a clinical trial. The oxazolidinedione derivatives are disclosed in Miller and Gutowski, (Patent RE 30,560, reissued March 31, 1981).

A second position on the molecule which has been modified is C—4'. A majority of these modifications have been based on the 3',4'-anhydro derivative, markeable both by coupling vindoline and catharanthine via a modified Polonovski reaction [Potier et al. *J.C.S. Chem. Comm.*, 670, (1975)] and by dehydrating VLB or leurosidine (Gutowski and Miller, Patent 4,029,663). The dehydration reaction produces two exo-double bond isomers in addition to the delta 3',4'-anhydro derivative. Functionalization of any one of these double bonds to form epoxides, diols, etc. has been the basis of chemical modifications at C—4'.

The third position of the indole-dihydroindole which has been modified successfully is C—4. In the first place, hydrolysis of the acetoxy group, present in all the above vinca alkaloids, yields active antineoplastic 4-desacetyl derivatives. (Vindesine, a C—3 carboxyamide, is a 4-desacetyl derivative.) Secondly, Hargrove (Patents 3,387,001 and 3,392,173) prepared novel 4-acyl derivatives of 4-desacetyl VLB, 4-desacetyl vincristine, etc. Among these new derivatives was 4-chloroacetyl VLB, which compound could be reacted wit amines, for example, dimethylamine, to yield a potent anticancer drug, vinglycinate, N,N-di-methyl-4-glycinyl VLB. In a different modification, Write and Neuss (Patent 4,122,082) oxidized the 4-hydroxyl of 4-desacetyl VLB to a 4-keto compound, and Thompson (Patent 4,195,022) reduced this ketone to the 4-epihydroxy (4α-hydroxy) derivative, also a compound with anticancer activity.

Indole-dihydroindole bridged dimers; i.e., the same or different alkaloid moieties bridged through the 3-carboxyl via a bis-amide are described in Conrad and Gerzon (Patent 4,199,504). Otherwise, indole-dihydroindole vinca alkaloid dimers have not been bridged through other positions in the molecule to form vinca tetramers.

VLB and vincristine have been conjugated with proteins to form materials useful in radioimmune assays. 4-Desacetyl VLB 3-carboxazide (desaceryl vinblastinoic azide) and the corresponding vincristine compound have been the derivatives employed) see Conrad et al., *J. Med. Chem.*, *22*, 391 (1979). And EP—A—O 041935, and Patent 4,203,898 for illustrations of this reaction.

This invention provides compounds of the formula I

$$R—O—CO—X—CO—Z \quad (I)$$

wherein R is a dimeric indole-dihydroindole radical derived from a 4-acetoxy or 4-hydroxy antineoplastic dimeric indole-dihydroindole alkaloid;

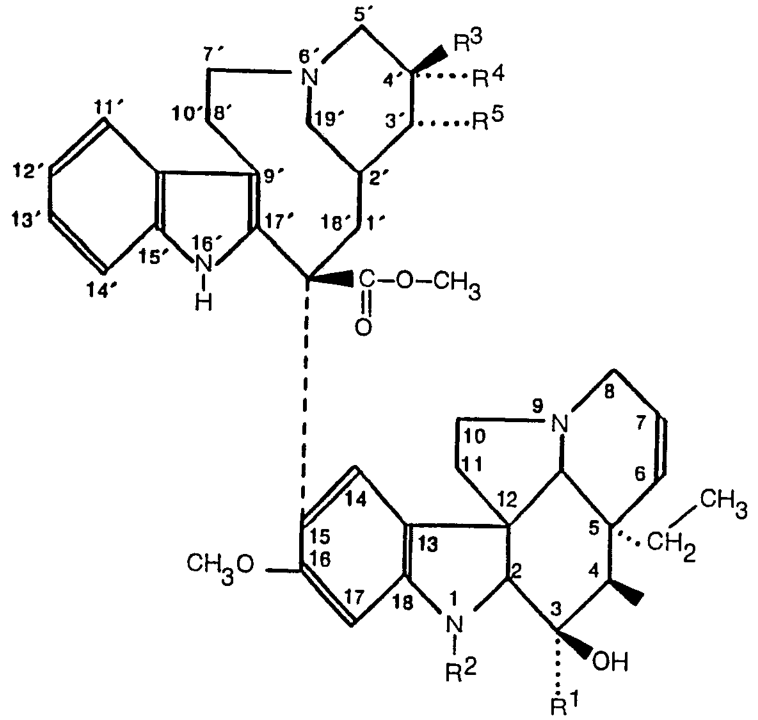

(II)

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbon atoms to which they are attached, they form an oxirane ring, and $R^3$ is ethyl; wherein $R^1$ is COOH, $COOC_{1-3}$alkyl or CO—$R^6$, where $R^6$ is $NH_2$, NH—$C_{1-3}$alkyl, NH—$CH_2CH_2Cl$, 1-pyrrolidyl, 1-piperidinyl, NH—$CH_2CH_2YCH_3$ wherein Y is S or O or a carboxy protecting group; wherein Z is OH, $OC_{1-3}$alkyl, $NH_2$, $NHNH_2$, or a carboxy activating group ($Z^1$) or a carboxy protecting group ($Z^2$); and wherein X is $C_{1-4}$ straight chain alkylene, $C_{2-8}$ branched alkylene, $C_{2-4}$alkylene, $C_{3-4}$alkynylene, $C_{3-6}$cycloalkylene, phenylene, hydroxy-substituted $C_{1-4}$alkylene or a direct bond; and salts thereof.

The compounds of this invention in which Z is formula (I) is OH, O—$C_{1-3}$alkyl, $NH_2$ or $NHNH_2$ have utility as antitumor compounds in transplanted tumors in mice, and also possess antimitotic properties.

When Z in formula I is a carboxy activating (acylating) group it can be any of the well known groups employed in the chemical art and in particular those used in peptide chemistry Such groups are well known in the art and are discussed for example, in Peptide Synthesis by M. Bodanszky, Y. S. Klausner and M. A. Ondetti, Second Edition (1976) John Wiley & Sons, notably pages 85 to 136. Amongst the values Z may take, there may be mentioned an azide (—$N_3$) group, a halogen atom for example bromine and especially chlorine, an acyloxy group of the formula $R^7$CO.O where $R^7$ is an aliphatic or aromatic residue such as for example $C_{1-3}$ alkyl, an alkoxy group preferably $C_{1-3}$ alkoxy or an aryloxy group, a methanesulphonyloxy, tosyloxy or benzenesulphonyloxy group, an imidazolyl radical or the residue of an N-acylhydroxylamine derivative, for example where Z is succinimidoxy, phthalimidoxy or benzotriazolyloxy.

When Z is a carboxy protecting group or $R^1$ is $COR^6$ and $R^6$ is a carboxy protecting group, it can be any of the well known groups employed for this purpose. This term refers to the commonly used carboxylic acid protecting groups employed to block or protect the carboxylic acid functionality while reactions involving other functional sites of the compound are carried out. Such carboxy protecting groups are noted for their ease of cleavage by hydrolytic or by hydrogenolytic methods to the corresponding carboxylic acid. Examples of carboxylic acid ester protecting groups are those of formula $OR^7$ where $R^7$ is *tert*-butyl, benzyl, 4-methoxybenzyl, $C_{2-6}$alkanoyloxymethyl, 2-indoethyl, *p*-nitrobenzyl, diphenylmethyl (benzhydryl), phenacyl, 4-halophenacyl, dimethylallyl, 2,2,2-trichloroethyl, tri($C_{1-3}$alkyl)silyl or succinimidomethyl. Other known carboxy protecting groups such as those described by E. Haslam in "Protective Groups in Organic Chemistry", Chapter 5, shall be recognized as suitable.

Specific examples of such carboxy activating or protecting groups include Cl, Br, $N_3$, imidazolyl, succinimidoxy

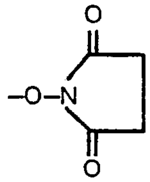

phthalimidoxy

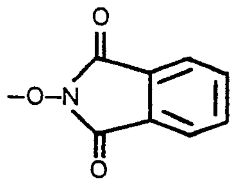

benzotriazolyloxy

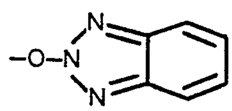

methanesulfonyloxy, tosyloxy or benzenesulfonyloxy, acylating groups ($Z^1$) or $CCl_3CH_2O$—, $CBr_3CH_2O$—, $CH_2ICH_2O$—, benzyloxy, methylbenzyloxy, t-butyloxy, allyloxy, methoxybenzyloxy, nitrobenzyloxy, phenacyloxy, nitrophenacyloxy, methoxyphenacyloxy, methylphenacyloxy, diphenylmethoxy, trityloxy (triphenylmethoxy), trimethylsilyloxy or the like carboxy protecting groups ($Z^2$).

Groups illustrative of X include methylene, ethylene, propylene, butylene, vinyl, propenylene, butenylene, butynylene, ethynylene, hydroxyethylene, 1,2-dihydroxyethylene, 1,2-dimethylethylene, 1,2,3,4-tetrahydroxybutylene, 3,4-dimethylbutylene, 1,4-cyclohexylene, 1,4-phenylene or 1,2-phenylene. Preferably X is $C_{1-4}$ alkylene, $C_{2-4}$ alkenylene, $C_{2-4}$ alkynylene, $C_{3-6}$ cycloalkylene or phenylene and is especially $C_{1-4}$ alkylene.

Salts of the compounds of this invention include cationic salts of the C—3 carboxylic acid group when $R^1$ is COOH or Z is OH, for example, sodium, potassium or tetramethyl ammonium. Also pharmaceutically-acceptable addition salts are included such as salts derived from non-toxic inorganic acids such as: hydrochloric acid, nitric acid, phosphoric acid, sulfuric acid, hydrobromic acid, hydriodic acid or phosphorous acid, as well as salts derived fron non-toxic organic acids such as aliphatic mono and dicarboxylic acids, phenyl-substituted alkanoic acids, hydroxyalkanoic and hydroxyalkandioic acids, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorbenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, naphthalene-1-sulfonate, naphthalene-2-sulfonate or mesylate.

The synthesis of the compounds of this invention can be carried out in stepwise fashion. First, a 4-desacetyl indole-dihydroindole of the formula ROH prepared by the procedure of Hargrove, U.S. Patent 3,392,173 is acylated with a carboxylic acid anhydride of the formula

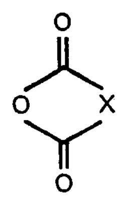

wherein X has its previous meaning, to yield a compound of formula I wherein Z is OH and R and X have their previous meanings. Compounds wherein Z is O—$C_{1-3}$ alkyl are prepared from the half-acid, R—CO—X—COOH, via one of the usual esterification procedures using a $C_{1-3}$alkanol. Methanol is the

preferred alkanol since the other ester groups present in starting materials of the formula ROH are methyl esters and transesterification problems are thus largely avoided.

When an indole-dihydroindole is to be reacted with e.g. succinic anhydride to prepare a compound of formula I wherein $R^1$ is COOH or $COR^6$ where $R^6$ is a carboxy protecting group, the C—3 ester group as well as the C—4 ester group must be hydrolyzed initially to yield, for example from VLB, a 4-desacetyl vinblastinoic acid — see U.S. Patent 4,012,390. Next, the C—3 carboxyl group must be protected with a carboxy protecting group as defined above. This C—3 carboxy protected derivative having a free hydroxyl at C—4 is then reacted as above with an anhydride. The resulting compound can then be manipulated chemically to yield compound according to formula I in which Z is an acylating moiety provided reaction conditions are neutral or basic, thus avoiding removal of the C—3 carboxy protecting group. After the desired terminal group, Z, is in lace, the carboxy protecting group at C—3 can be removed to yield compounds according to formula I in which $R^1$ is COOH.

Alternatively, compounds of the formula

$$R—O—CO—X—CO—OC_{1-3}alkyl$$

can be prepared by using a half ester, half acid chloride as the acylating agent: i.e., $Cl—CO—X—CO—O—C_{1-3}alkyl$. Other acylating groups can be used in place of Cl, and the acylating moiety can be represented generally by the formula

$$Z^1—CO—X—CO—OC_{1-3}alkyl$$

wherein X has its previous meaning and $Z^1$ is Cl, Br, $N_3$, succinimidoxy, phthalimidoxy, methanesulfonyloxy, tosyloxy, phenylsulfonyloxy, benzotriazolyloxy, or other acylating moiety. Alternatively, an acylating agent of the formula $Z^1—CO—X—CO—Z^2$ where $Z^2$ is a carboxy protecting group, can be used to yield a compound of the formula $R—O—CO—X—CO—Z^2$.

Compounds according to formula I in which Z is $NH_2$ or $NHNH_2$ are prepared by forming an "activated" vinca dimer (R group) 4-hemi acid of the formula

$$R—O—CO—X—CO—Z$$

where Z is preferably Cl, with ammonia or hydrazine. A mixed anhydride is formed from the half-acid by treatment successively with N-methylmorpholine and an alkyl chloroformate. Reaction of the mixed anhydride with alcoholic ammonia or hydrazine yields the desired half-amide. If a compound is to be prepared in which $R^1$ is COOH and Z is $NH_2$ or $NHNH_2$, standard basic reactions can be employed provided the $R^1$ carboxyl group is first protected.

Compounds in which Z is OR in which R is also an indoledihydroindole radical represented by II are prepared by forming an acylating moiety of the formula

$$R—O—CO—X—CO—Z^1$$

and reacting it with the same or different 4-desacetyl indoledihydroindole alkaloid, ROH, provided any free carboxyl group at C—3 is first protected, such protecting group being optionally removed when the reaction is complete.

Alternative procedures for preparing several of the above derivatives involve the use of a coupling agent such as a carbodiimide, for example, DDC (dicyclohexylcarbodiimide) or EEDQ (N-ethoxycarbonyl-2-ethoxy-1,2-dihydroquinoline), under anhydrous reaction conditions with a half-acid $HO—CO—X—CO—Z^2$, where $Z^2$ is a carboxy protecting group. For example, an initial 4-succinoxy derivative can be prepared from ROH and $HO—CO—CH_2—CH_2—CO—Z^2$ in the presence of DCC to yield a compound of the formula

$$R—O—CO—X—CO—Z^2.$$

The carboxy protecting group can then be removed and the resulting free acid reacted with hydroxyphthalimide, hydroxybenzotriazole, hydroxysuccinimide, halogenating agent or azide to yield reactive acylating intermediates of the formula

$$R—O—CO—X—CO—Z^3$$

wherein $Z^3$ is succinimidoxy, benzotriazolyloxy, phthalimidoxy, Cl, Br, $N_3$. These intermediates are then reacted with a second, same or different, indole-dihydroindole ROH, to form a bridged vinca tetramer or can be reacted with immunoglobulin or fragments of immunoglobulin to form conjugates, for example, useful in a radioimmune assay or with polyclonal or monoclonal antibodies to yield anticancer drugs. In

addition the "activated" derivatives can be reacted with a lower alcohol to yield half esters of the formula

$$R—O—CO—X—CO—O—C_{1-3}alkyl$$

When X is the compound to be prepared is hydroxy, dihydroxy or tetrahydroxy $C_{1-4}$alkylene; i.e., a linking group derived from malic, tartaric acid or saccharic acid, in preparing the compounds it is necessary to protect the hydroxy or hydroxyls with a protecting group such as a pyranyl group. A trialkylsilyl group such as a trimethylsilyl group can also be used. In the instance of a vicinal dihydroxy compound such as tartaric acid, an acetal; i.e., an isopropylidene or cyclohyeoxylidene derivative, can be used.

When X is a direct bond (the linking group is formed from oxalic acid), oxalylchloride cannot be used since the hemioxalate may cyclize with the 3-hydroxyl. However, an oxalate half ester or an oxalic acid derivative of the formula Cl—CO—CO—$Z^2$ can be used and the ester hydrolyzed or the carboxy protecting group removed during the simultaneous conversion to an acylating moiety, R—CO—CO—$Z^1$.

Generally, the compounds of this invention of the structure R—O—CO—X—CO—$Z^1$ where $Z^1$ is Cl, Br, tosyloxy, benzenesulfonyloxy, methanesulfonyloxy, $N_3$ or other acylating moiety are useful not only in preparing those compounds, of this invention wherein R and Z both contain indole-dihydroindole alkaloid radicals, but as stated above, are also useful for coupling to immunoglobulin such as polyclonal and monoclonal antibodies so as to provide suitable conjugates for radioimmune assay purposes or with anticancer properties.

Such conjugates are prepared by reacting the polyclonal or monoclonal antibody with the compound of structure R—O—CO—X—CO—$Z^1$ under conventional conditions such as for example in aqueous medium and at a temperature of from 5°C to 25°C, for example at room temperature, and at a pH of 7.5 to 9.5, preferably 8.0 to 9.0. The process results in the attachment by convalent linkage of one or more vinca residues at the free amino groups of the immunoglobulin molecule, for example, amino groups derived from lysine residues. The number of residues attached will depend on the concentration of the reactants and the duration of the reaction but the average number is usually for example from 3 to 14 or 20.

For example in carrying out the reaction, a solution of the compound of formula R—O—CO—X—CO—$Z^1$ in a suitable solvent such as dioxan is slowly added dropwise to a buffered solution of immunoglobulin in for example 0.34 *M* borate buffer at pH 8.6. The conjugate is isolated by gel filtration and stored in saturated ammonium sulphate solution being readily brought back into solution by dialysis with a buffer solution for example a phosphate buffered saline pH 7.4, or alternatively it can be stored in a refrigerator at 4°C or frozen at for example −20°C.

The preferred materials for preparing such conjugates are monoclonal or polyclonal antibodies to human or animal tumor associated antigens such as for example

(i) Ig from goats or sheep immunised with carcinoembryonic antigen
(ii) Ig from rabbit antiacute lymphoblastic leukemia serum.
(iii) Ig from various primate antisera raised against acute lymphoblastic leukemia, acute myleoblastic leukemia, chronic lymphoblastic leukemia and chronic granulocytic leukemia
(iv) Ig from goats or sheep immunized with lung carcinoma material
(v) monoclonal Ig from mouse hybridomas secreting anti-human colorectal carcinoma antibodies
(vi) monoclonal Ig from mouse hybridomas secreting anti-human melanoma antibodies
(vii) monoclonal Ig from mouse hybridomas secreting antibodies reacting with human leukemia cells
(viii) monoclonal Ig from mouse hybridomas secreting antibodies reacting with human neuroblastoma cells
(ix) monoclonal Ig from mouse hybridomas secreting antibodies reacting with human breast cancer antigens
(x) monoclonal Ig from mouse hybridomas secreting antibodies reacting with human ovarian carcinoma cells
(xi) monoclonal Ig from mouse hybridomas secreting antibodies reacting with human osteosarcoma cells
(xii) monoclonal Ig from mouse hybridomas secreting antibodies to lung carconoma.

Starting 4-desacetyl indole-dihydroindole alkaloids and ROH useful in forming the compounds of this invention, can be represented by the following 2-dimensional structure

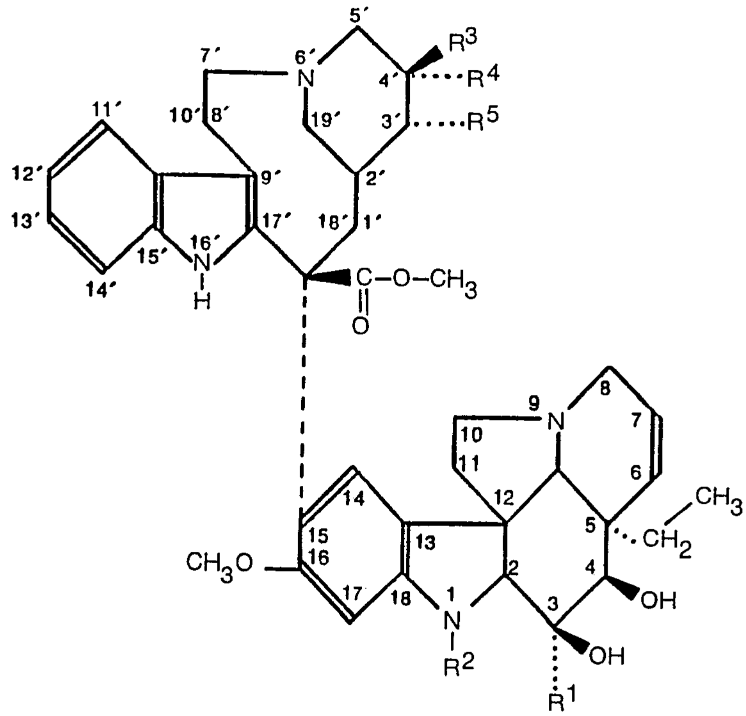

(III)

wherein $R^2$ is H, $CH_3$ or CHO; when $R^4$ and $R^5$ are taken singly, $R^5$ is H and one of $R^3$ and $R^4$ is ethyl and the other is H or OH; when $R^4$ and $R^5$ are taken together with the carbon atoms to which they are attached, they form an oxirane ring, and $R^3$ is ethyl; and $R^1$ is COOH, $COOC_{1-3}$alkyl, or $COR^6$ wherein $R^6$ is $NH_2$, NH—$C_{1-3}$alkyl, NH—$CH_2CH_2Cl$, 1-pyrrolidyl, l-piperidinyl or NH—$CH_2CH_2YCH_3$ wherein Y is S or O.

In the above formula, where $R^1$ is CO—$OCH_3$, $R^2$ is methyl, $R^3$ is hydroxyl, $R^4$ is ehtyl and $R^5$ is H, 4-desacetyl VLB (4-desacetyl vinblastine) is represented; where $R^1$ is CO—$OCH_3$, $R^2$ is formyl, $R^3$ is hydroxyl, $R^4$ is ethyl and $R^5$ is H, 4-desacetyl vincrisine is represented; where $R^1$ is CO—$OCH_3$, $R^2$ is methyl, $R^3$ is ethyl, $R^4$ is hydroxyl, and $R^5$ is H, 4-desacetyl leurosidine is represented; where $R^1$ is CO—$OCH_3$, $R^2$ is methyl or formyl, $R^3$ is ethyl and $R^4$ and $R^5$ taken together with the carbon atoms to which they are attached form an α-epoxide ring, 4-desacetyl leurosine and 4-desacetyl leuroformine, respectively are represented; where $R^1$ is CO—$OCH_3$, $R^2$ is methyl, $R^3$ is ethyl, $R^4$ and $R^5$ are H, 4-desacetyl deoxy VLB "B" or 4-desacetyl-4'-deoxy-leurosidine or 4-desacetyl-4'-epideoxy VLB is represented; where $R^1$ is CO—$OCH_3$, $R^2$ is methyl, $R^4$ is ethyl and $R^3$ and $R^5$ are H, 4-desacetyl deoxy VLB "A" or 4-desacetyl-4'-deoxy VLB is represented; where $R^1$ is CO—$OCH_3$, $R^2$ is CHO, $R^3$ is ethyl, $R^4$ and $R^5$ are H, 4-desacetyl-4'-epideoxyvincristine (4-desacetyl-1-formyl-1-desmethyl-4'-deoxyleurosidine) is represented; and where $R^1$ is CO—$NH_2$, $R^2$ is methyl, $R^3$ is OH, $R^4$ is ethyl and $R^5$ is H, vindesine (4-desacetyl-VLB 3-carboxamide) is represented. Other 3-carboxamide derivatives of the desacetyl indole-dihydroindole alkaloids represented by III are named accordingly; i.e. as the 3-(2-methylthio)ethyl carboxamide derivative, as the 3-(2-methoxy)ethylcarboxamide, as the 3-carboxhydrazide, as the 3-pyrrolidinyl derivative, as the N-methylcarboxamide derivative, for each of the amide groups comprehended within $R^1$ above. Compounds according to II in which $R^1$ is a carboxyl group are named and "oic acids", i.e., 4-desacetyl vinblastinoic acid, 4-desacetyl leurosinoic acid, 4-desacetyl vincristinoic acid, etc. With regard to formation of derivatives of 4-desacetyl vinblastinoic acid, it will be appreciated by those skilled in the art that the 3-carboxyl must be protected with one of the carboxy protecting groups, such as those listed above in defining $Z^2$, prior to reaction with an anhydride or other acylating agent, $Z^1$—CO—X—$COOC_{1-3}$alkyl, and that the product of this reaction R—O—CO—X—$COOC_{1-3}$alkyl, must be hydrolyzed and converted to a hemiacid carrying an acylating group, ($COZ^1$) i.e., COCl, COBr, CO—$N_3$, succinimidoxy, without use of a second carboxy protecting group.

Literature references to the parent alkaloids of the 4-desacetyl derivatives III are as follows: leurosine (U.S. Patent No. 3,370,057), VLB (U.S. Patent No. 3,097,137), leurosidine (vinrosidine) and leurocristine (to be referred to hereinafter as vincristine) (both U.S. Patent No. 3,205,220), desmethyl VLB (U.S. Patent No. 3,354,163), vindesine and other 3-carboxamides (U.S. Patent No. 4,203,898), vinblastinoic acid, vincristinoic acid, etc. (U.S. Patent No. 4,012,390), 4'-epivincristine (U.S. Patent No. 4,143,041) leuroformine, formylleurosine (U.S. Patent No. 4,279,816), and deoxy VLB "A" and "B" [*Tetrahedron Letters*, 783 (1958)].

The preparation of typical compounds according to formula II is illustrated below. For ease of naming, a compound of the structure R—O—CO—$CH_2$—$CH_2$—COOH, for example, would be designated a VLB-4-hemisuccinate, omitting the 4-desacetyl term as common to all R radicals.

Example 1

Preparation of VLB-4-Hemisuccinate

Two grams of 4-desacetyl VLB were dissolved in pyridine to which solution were added 2 g of succinic anhydride. The reaction mixture was stirred at ambient temperature for 5 hours. (Temperatures in the range 0—50°C may be used for this reaction.) the volatile constituents were removed by evaporation *in vacuo* and the residue taken up in $CH_2Cl_2$. The $CH_2Cl_2$ layer was washed with 5% aqueous sodium bicarbonate, and then with water. The organic layer was dried and the solvent removed therefrom *in vacuo*. VLB 4-hemisuccinate thus prepared had the following physical characteristics:

IR: peaks at 1737, 1615, 1460, 1434 $cm^{-1}$

nmr: ($CDCl_3$) 8.05, 7.54, 7.14, 6.58, 6.11, 5.83, 5.46, 5.28, 3.80, 3.78, 3.69, 3.62, 2.71, 0.92, 0.79 ppm.

The sulfate salt was prepared by dissolving VLB hemisuccinate in anhydrous ethanol and 2% ethanolic sulfuric acid added to pH = 3.95, and then evaporating the volatile constituents. The sulfate salt had the following physical characteristics:

U.V. ($H_2O$) maximum at 214, 268, 283, 312 nm

IR (KBr): peaks at 3400 (broad), 1740 $cm^{-1}$

Titration (66% DMF): pKa = 4.80, 6.10, 7.80

The above procedure was used to prepare the following additional compounds:

Vincristine 4-hemisuccinate from 4-desacetylvincristine; yield = 700 mg (from 1.95 g). The compound had the following physical charcteristics:

IR: peaks at 1740, 1684 $cm^{-1}$

nmr ($DCCl_3$): 8.77, 8.15, 8.11, 7.72, 7.54, 7.18, 6.90, 6.83, 5.89, 5.39, 5.21, 4.69, 4.51, 3.86, 3.74, 3.67 ppm

The sulfate salt was prepared by adding 2% ethanolic sulfuric acid to an ethanol solution of the free base (400 mg);

yield = 330 mg; $R_f$ (silica gel, methanol) = 0.16.

Vindesine 4-hemisuccinate was prepared from 300 mg of vindesine (4-desacetyl VLB C—3 carboxamide); yield = 290 mg. The compound had the following physical characteristics:

IR: peaks at 3450, 1733, 1693 $cm^{-1}$

nmr ($CDCl_3$): 8.07, 7.52, 7.10, 6.54, 6.08, 5.92, 5.49, 5.27, 3.70, 3.59, 3.46, 2.83, 0.91, 0.78 ppm.

The sulfate salt was prepared as above (200 mg of free base gave 160 mg of a white amorphous powder) tlc $R_f$ (silica gel, methanol) = 0.56.

4'-epideoxy VLB 4-hemisuccinate from 4-desacetyl-4'-epideoxy VLB (1080 mg); yield = 540 mg; $R_f$($SiO_2$, 1:1 EtOAc/MeOH) = 0.08.

Vinblastinoic acid 4-hemisuccinate from 4-desacetyl vinblastonoic acid. The compound had the following physical characteristics:

$R_f$($SiO_2$ gel, MeOH) = 0.23

nmr ($CDCl_3$): 8.05, 7.52, 7.11, 6.57, 6.06, 5.71, 5.26, 5.14, 3.75, 3.60, 2.82, 0.90, 0.76 ppm.

Following the above procedure, 4-desacetyl VLB was reacted with maleic anhydride to form VLB 4-hemimaleate. The compound had the following physical characteristics:

IR: peaks at 1730, 1590 $cm^{-1}$

nmr: ($CHCl_3$) 8.61, 8.04, 7.50, 7.12, 6.59, 6.48, 5.78 (J = 12Hz) 6.09, 5.7, 5.51, 5.3, 3.79, 2.70 ppm.

Following the above procedure, VLB 4-hemiglutarate was prepared (700 mg from 3 g starting material) with the following physical characteristics:

IR: peak at 3450, 1736 $cm^{-1}$

nmr: ($CDCl_3$) 8.07, 7.53, 7.13, 6.53, 6.13, 5.83, 5.45, 5.24, 3.80, 3.68, 3.63, 2.69, 0.91, 0.81 ppm.

$R_f$($SiO_2$, 1:1 EtOAc/MeOH) = 0.25 sulfate salt (yield = 50%)

$R_f$($SiO_2$, 1:1 EtOAc/MeOH) = 0.08.

In any of the above acylations of a 4-desacetyl indoledihydroindole vinca dimer, any incidental acylation of the 3—OH can be reversed by treatment with wet silica gel according to the procedure of Hargrove, U.S. Patent 3,392,173. Alternatively, the compounds can be purified from any 3-acyl derivative or other by-products of the reaction by chromatography, conveniently over silica gel using an ethyl acetate/methanol solvent mixture as the eluant.

Example 2

Preparation of "Activated" VLB 4-Hemisuccinate and Conjugate

Ninety mg of VLB 4-hemisuccinate were dissolved in 2 ml of $CH_2Cl_2$. Fifteen µl of N-methyl morpholine were added and the resulting solution cooled to about 0°C. About 20 µl of isobutyl chloroformate were added followed by 20 mg of N-hydroxysuccinimide. The reaction mixture was heated to reflux for about 15 minutes, and the solvent and other volatile constituents removed by evaporation in vacuo. The gummy residue was used for conjugation with proteins without further purification. The compound had the following structure:

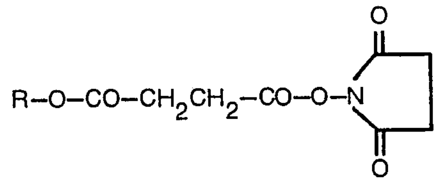

where R is a VLB radical as set forth above. The compound can be named systematically as 4-[3-(1-succinimidooxycarbonyl)]propionyl VLB. It has the following physical characteristics:
IR: ($CHCl_3$) peaks at 1741, 1718 $cm^{-1}$

In a separate run, the intermediate mixed anhydride of VLB 4-hemisuccinate and isobutylcarbonic acid, having the structure below, was isolated and characterized as follows:
IR: peaks at 3450, 1738, 1820 $cm^{-1}$
nmr ($CDCl_3$): 8.05, 7.45, 7.15, 6.40, 6.10, 5.88, 5.42, 5.37, 4.00, 3.81, 3.76, 3.65, 2.74, 0.95 ppm.

$$R—O—CO—CH_2—CH_2—CO—O—CO—O\text{-isobutyl}$$

where R is VLB linked to the mixed anhydride function at C—4.

350 µl of 14.7 mg/ml solution of "activated" VLB 4-hemisuccinate in DMF was added with rapid stirring to 2.0 ml of a 20.0 mg/ml solution of mouse monoclonal anti-lung small cell carcinoma antibody in 0.34 M borate buffer pH 8.6. After stirring at room temperature for 4 hours the reaction mixture was adjusted to pH 7.4 using 1N HCl and clarified by centrifugation. The product was isolated by gel filtration on a 2.0 × 22.0 cm (67.0 ml) column of Bio-Gel P—6 equilibrated with phospahte buffered saline. The excluded peak was collected (9.7 ml) and assayed for desacetylvinblastine and protein by spectrometry at 270 and 280 nm. The conjugate so prepared contained 7.5 moles VLB per mole of Ig.

An improved method of preparing "activated" VLB 4-hemisuccinate where the activating group is the 1-succinimidoxy group is as follows.

One gram of VLB 4-hemisuccinate was mixed with 380 mg of N-methylmorpholine in 20 ml of methylenedichloride, and 390 mg of isobutylchloroformate were added. The reaction mixture was stirred at about 0°C under a nitrogen atmosphere for about 45 minutes. 795 mg of N-hydroxysuccinimide were added and the reaction mixture heated at reflux temperature under $N_2$ with stirring for about 45 minutes. The rection mixture was cooled and the cooled mixture washed with dionized water and then dried immediately with $Na_2SO_4$. The drying agent was separated by filtration and the filtrate evaporated to dryness in vacuo; residue weight = 900 mg; tlc indicated 90+% purity.

Following the original procedure, VLB 4-hemiglutarate was treated successively with N-methylmorpholine, isobutylchloroformate and N-hydroxysuccinimide in methylenechloride solution to yield 160 mg of 4-[4-(1-succinimidoxycarbonyl)]butyryl VLB from 400 mg of 4-glutaryl VLB.

Following the above procedure, 4'-epideoxy VLB 4-hemisuccinate wsa converted to 4-[3-(1-succinimidoxycarbonyl)]propionyl VLB. Chromatography over $SiO_2$ gel using 1:1 EtOAc/MeOH; $R_f$ = .23; yield = 360 mg from 540 mg of starting hemisuccinate.

Following the above procedure, "activated" vindesine 4-hemisuccinate or 4-[3-(1-succinimidoxy-carbonyl)]propionyl vindesine was preapred. The compound had the following physical characteristics: IR maxima at 3520, 3470, 3400, 1810, 1791, 1744 with a broad shoulder 1744—1650 $cm^{-1}$.
nmr ($CDCl_3$): 8.08, 7.45, 7.15, 6.44, 6.12, 5.85, 5.48, 5.32, 3.79, 3.64, 3.58, 2.85, 2.84, 0.95, 0.78 ppm.

Also prepared was 4-[3-(1-succinimidoxycarbonyl)]propionyl vincristine; yield = 140 mg from 256 mg of starting material.
IR peaks at 3460, 1810, 1785, 1744, 1718 and 1683 $cm^{-1}$.
nmr ($CDCl_3$): 8.79, 8.19, 8.14, 7.78, 7.41, 7.18, 6.97, 6.84, 5.92, 5.42, 5.35, 4.72, 4.52, 3.81, 3.78, 3.71, 2.85, 0.83 ppm.

## Example 3

Preparation of Methyl VLV 4-Hemisuccinate

200 mg of VLB 4-hemisuccinate were dissolved in 10 ml of acetic anhydride. Five ml of glacial acetic acid were added followed by 200 ml of methanol containing five drops of pyridine. The solution was cooled for one-half hour and then allowed to remain at room temperature for 16 hours. Evaporation of the volatile constituents resulted in an oil. The residue was dissolved in water and the aqueous solution made basic by the addition of 14N aqueous ammonium hydroxide. The basic aqueous layer was extracted with $CH_2Cl_2$. The $CH_2Cl_2$ extract was washed with water and dried. Removal of the solvent left the methyl ester of VLB 4-succinate as a residue (also named as 4-(3-methoxycarbonyl)propionyl VLB.

An improved method of preparing the half methyl ester follows:

1020 mg of VLB 4-hemisuccinate activated with N-hydroxysuccinimide, as provided by Example 2, were dissolved in 25 ml of MeOH. The reaction was sealed under $N_2$ and protected from the light. After 18 hours, the volatile constituents were removed in vacuo. The residue was chromatographed on an HPLC silica gel column eluted with a gradient of EtOAc to EtOAc-MeOH (1:1). Fractions containing the desired

product, as determined by tlc, were combined. Volatile constituents were removed in vacuo yielding 310 mg of methyl-4-hemisuccinate VLB as a tan amorphous powder. The sulfate was prepared in the usual manner. (2% $H_2SO_4$ in 2BEtOH).

Other methyl esters prepared by the above procedure include:
Methyl vindesine 4-hemisuccinate
$R_f$($SiO_2$, 1:1 EtOAc/MeOH) = 0.5
IR: peaks at 1735, 1699 $cm^{-1}$
Mass spectrum: 867 (M+), 836 (M—31), 808 (M—59)
nmr ($CDCl_3$): 9.94, 8.05, 7.52, 7.14, 6.98, 6.58, 6.12, 5.87, 5.53, 5.30, 3.79, 3.69, 3.62, 3.47, 2.74, 0.90, 0.81 ppm.
Sulfate salt: $R_f$($SiO_2$, 1:1 EtOAc/MeOH) = 0.55 Methy 4'-epideoxy VLB 4-succinate
Mass spectrum: 866 (M+), 864, 880, 894, 908, 339 (M-vidoline moiety), 139
IR: peaks at 1743 $cm^{-1}$
nmr ($CDCl_3$): 8.03, 7.55, 7.16, 6.60, 6.10, 5.89, 5.46, 5.39, 3.82, 3.72, 3.64, 2.76

Example 4

Preparation of 4-Succinoyl VLB Amide

One gram of VLB 4-hemisuccinate was dissolved in about 25 ml of methylene dichloride. 200 mg of N-methyl morpholine were added to the solution under a nitrogen atmosphere while the reaction mixture was cooled in an ice bath. 200 mg of isobutylchloroformate were added and the reaction mixture stirred at room temperature at about 0°C for 15 minutes. The reaction mixture was then evaporated to dryness to yield a tan gum. The gum was dissolved in methanolic ammonia and kept at ambient temperature under an $N_2$ atmosphere for about 48 hours. Evaporation of the volatile constituents yielded a residue comprising the amide (on the 4-suiccinate), of VLB 4-hemisuccinate, named as 4-succinoyl VLB amide for convenience. the amide had the following physical characteristics:
IR peaks at 1738, 1685 $cm^{-1}$
Mass Spectrum: 867 (M+), 355, 154
nmr ($CDCl_3$): 9.86 8.04, 7.53, 7.12, 6.63, 6.10, 5.85, 5.48, 5.32, 3.81, 3.80, 3.73, 3.62, 2.72, 0.90, 0.82 ppm.
$R_f$($SiO_2$, 1:1 EtOAc/MeOH) = 0.38
Sulfate salt:
$R_f$($SiO_2$, 1:1 EtOAc/MeOH) = 0.33.

The corresponding hydrazide, 4-succinoyl VLB hydrazide, was prepared as above by using methanolic hydrazine and a greatly shortened reaction time. The compound had the following physical characteristics:
$R_f$($SiO_2$, 1:1 EtOAc/MeOH) = 0.23
IR: peaks at 3400, 3450, 1739, 1680 $cm^{-1}$
nmr ($CDCl_3$): 9.88, 8.03, 7.53, 7.12, 6.55, 6.10, 5.85, 5.46, 5.27, 3.79, 3.60, 2.30, 0.88, 0.81 ppm.

Example 5

Preparation of Methyl VLB 4-Adipoate

One and four-tenths grams of 4-desacetyl VLB were dissolved in 50 ml of methylene dichloride. Three grams of adipic acid were added followed by 3 g of dicyclohexylcarbodiimide. The reaction mixture was maintained in a water-free atmosphere at ambient temperature for about 24 hours. The reaction mixture was then filtered and the filter cake washed with methylene dichloride. Evaporation of the methylene dichloride yielded a residue which was purified by chromatography over silica gel using 1:1 ethyl acetate/methanol solvent mixture as the eluant. The major product of the chromatography was methyl VLB 4-adipoate, apparently produced by the presence of unreacted dicyclohexylcarbodiimide and methanol during chromatography; yield = 220 mg; nmr, peak at 3.63 ($CH_3O$— new methyl ester); mass spectrum; peaks at 910 (M+) 924 (M+14), 879, 852, 355, 154.

The following illustrates the scope of the compounds represented by II above. In naming these compounds, where a group present in the original indole-dihydroindole dimer has been replaced by a new function; i.e., 4-acetoxy replaced by 4-succinoxy or 3-methylcarboxylate by carboxamide, the group removed will be omitted. For example, VLB 4-succinate instead of 4-desacetyl VLB 4-succinate or vindesine 4-succinate for 4-desacetyl-3-desmethoxycarbonyl VLB 3-carboxamide 4-succinate:

4'-deoxy-4-[3-(1-succinimidoxy)propionyl VLB
4'-deoxy-4-(3-ethoxycarbonyl)propionyl-1-formyl leurosidine
4'-deoxy-1-formylleurosidine-4-maleate
4'-deoxy-1-formyl-4-(3-azidocarbonyl)propanoyl leurosidine
4-(4-t-butyloxycarbonyl)butynylleurosine
4-[5-(1-phthalimidoxy)]valerylvinblastinoic acid
4-(3-methoxycarbonyl)propiolyl vincristinoic acid
4-[3-(2-benzotriazolyloxy)]propionyl vincristine 3-(2-chloroethyl)carboxamide
4-[(3-trilyloxycarbonyl)propionyl leurosidine 3-(1-pyrrolidyl)carboxamide
4'-deoxy VLB 3-hemiglutarate
leurosine 3-hemiadipate
bis-[4-(4-desacetyl VLB)]succinate
bis-[4-(4-desacetyl VLB 3-carboxamide)]maleate

The compounds of this invention in which Z is OH, $NH_2$, $NHNH_2$, or O—$C_{1-3}$ alkyl have utility as antitumor compounds active against transplanted tumors in mice. Compounds in which Z is succinimidoxy, phthalimidoxy, Br, Cl, $N_3$, benzotriazolyloxy, tosyloxy, benzenesulfonyloxy or methanesulfonyloxy are useful, as previously stated, in the preparation of antibody conjugates.

As evidence of the utility of compounds according to I above in which Z is OH, $NH_2$, $NHNH_2$ or O—$C_{1-3}$ alkyl as mitotic inhibitors, there ability to cause metaphase arrest was measured by standard procedures. Table I gives the results of this study. In the table, column 1 gives the name of the compound, and column 2 the concentration in the medium of the compound from column 1 in mcg/ml showing metaphase arrest.

TABLE I

| Name of Compound | Concentration in mcg/ml Showing Metaphase Arrest |
|---|---|
| 4-Succinoyl VLB amide | 0.2 |
| Methyl VLB 4-hemisuccinate sulfate | 0.02 |
| Methyl vindesine 4-hemisuccinate sulfate | 0.2 |
| VLB 4-hemiglutarate sulfate | 0.2 |
| Vindesine 4-hemisuccinate sulfate | 0.2 |

Certain of the above compounds have also shown activity against transplanted tumors in mice. This information is summarized in Table II in which column 1 gives the name of the compound, column 2 the tumor, column 3 the dose level in mg/kg and column 4 the present tumor inhibitor. P1534J is a leukemia and 6C3HED a lymphosarcoma.

TABLE II

| Name of Compound | Tumor | Dose | Percent Inhibition |
|---|---|---|---|
| Vindesine 4-hemisuccinate | 6C3HED | 12 | 48 |
| | | 25 | 98 |
| | | 50 | 100 |
| VLB 4-hemisuccinate sulfate | P1534J | 18 | 57 |
| | | 36 | Toxic |
| | | 72 | Toxic |
| | 6C3HED | 18 | 100 |
| | | 36 | 100 |
| | | 72 | Toxic |
| Vincristine 4-hemisuccinate sulfate | P1534J | 20 | 63 |
| | | 40 | 83 |
| | | 60 | 94 |
| | | 80 | 96 |
| | 6C3HED | 20 | 100 |
| | | 40 | 100 |
| | | 60 | 100 |
| | | 80 | 100 |

**Claims for the Contracting States: BE CH DE FR GB IT LI LU NL SE**

1. A compound of the formula I:

R—O—CO—X—CO—Z (I)

wherein R has the formula I:

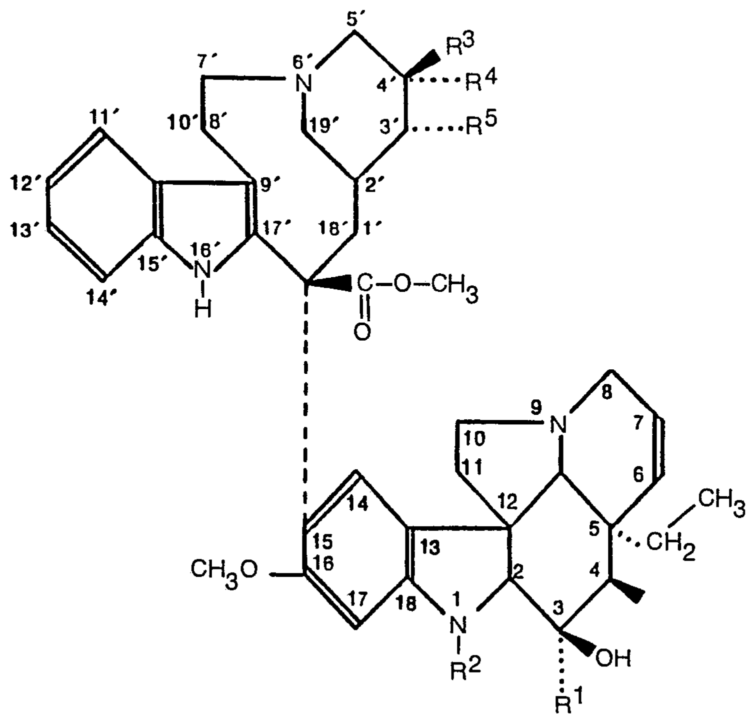

(II)

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbon atoms to which they are attached, they form an oxirane ring, and $R^3$ is ethyl; wherein $R^1$ is COOH, $COOC_{1-3}$alkyl or CO—$R^6$, wherein $R^6$ is $NH_2$, NH—$C_{1-3}$alkyl, NH—$CH_2CH_2Cl$, 1-pyrrolidyl, 1-piperidinyl, or NH—$CH_2CH_2YCH_3$ wherein Y is S or O; wherein Z is OH, $OC_{1-3}$alkyl, $NH_2$, $NHNH_2$, or a carboxy activating group ($Z^1$) or a carboxy protecting group ($Z^2$); and wherein X is $C_{1-4}$ straight chain alkylene, $C_{2-8}$ branched alkylene, $C_{2-4}$alkenylene, $C_{3-4}$alkynylene, $C_{3-6}$cycloalkylene, phenylene, hydroxy-substituted $C_{1-4}$alkylene or a direct bond; and salts thereof.

2. A compound according to claim 1 in which Z is OH, $OC_{1-3}$ alkyl, $Z^1$ or $Z^2$.

3. A compound according to claim 2 in which Z is OH or $OC_{1-3}$ alkyl.

4. A compound according to claim 2 in which Z is Br, Cl, $N_3$, succinimidoxy, phthalimidoxy, benzotriazolyloxy, methansulfonyloxy, tosyloxy or benzenesulphonyloxy.

5. A compound according to claim 2 in which Z is $CCl_3CH_2O$, $CBr_3CH_2O$, $CH_2ICH_2O$, benzyloxy, methylbenzyloxy, *t*-butyloxy, allyloxy methoxybenzyloxy, nitrobenzyloxy, phenacyloxy, nitrophenacyloxy, methoxyphenacyloxy, methylphenacyloxy, diphenylmethoxy, trityloxy, trimethylsilyloxy.

6. A compound according to any of claims 2 to 5 in which X is $C_{1-4}$ alkylene.

7. A pharmaceutical formulation comprising a compound as defined in claim 1 wherein Z is OH, $OC_{1-3}$alkyl, $NH_2$ or $NHNH_2$, and a pharmaceutically acceptable diluent or carrier.

8. A process for producing a compound as defined in claim 1 which comprises reacting a compound of formula

A—CO—X—CO—B

where either A is $Z^1$ and B is $Z^2$ or $OC_{1-3}$alkyl, or A and B together form an —O— link, with a 4-hydroxy dimeric indole-dihydroindole, ROH, and optionally removing a $C_{1-3}$ alkyl or $Z^2$ group to give the free acid in which Z is OH, or which comprises activating a compound of formula R—O—CO—X—COOH to yield a compound in which Z is $Z^1$ and optionally reacting the product with ammonia, hydrazine or a $C_{1-3}$ alkanol to yield a compound in which Z is $NH_2$, $NHNH_2$ or $OC_{1-3}$ alkyl.

**Claims for the Contracting State: AT**

1. A process for producing a compound of the formula I:

R—O—CO—X—CO—Z (I)

wherein R has the formula I:

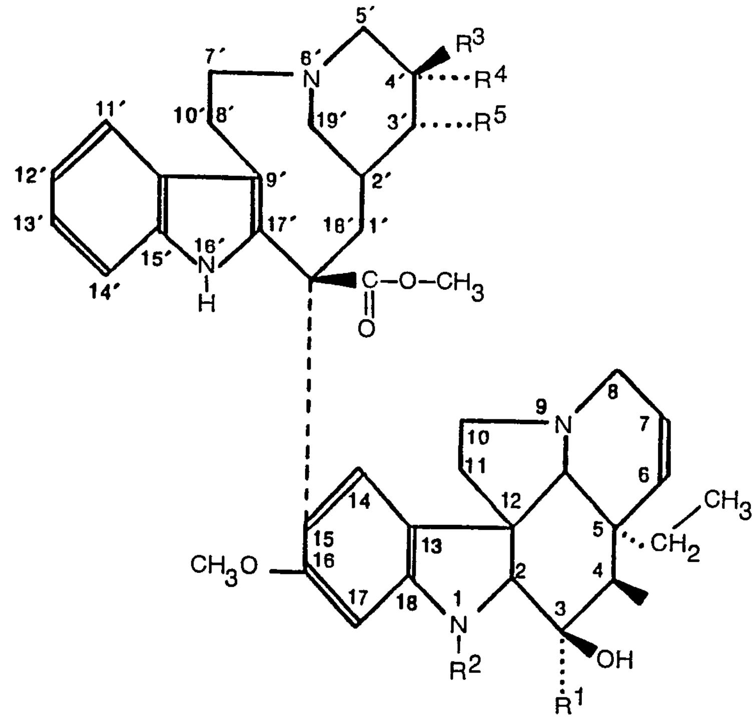

(II)

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbon atoms to which they are attached, they form an oxirane ring, and $R^3$ is ethyl; wherein $R^1$ is COOH, $COOC_{1-3}$alkyl or CO—$R^6$, wherein $R^6$ is $NH_2$, NH—$C_{1-3}$alkyl, NH—$CH_2CH_2Cl$, 1-pyrrolidyl, 1-piperidinyl, or NH—$CH_2CH_2YCH_3$ wherein Y is S or O; wherein Z is OH, $OC_{1-3}$alkyl, $NH_2$, $NHNH_2$, or a carboxy activating group ($Z^1$) or a carboxy protecting group ($Z^2$); and wherein X is $C_{1-4}$ straight chain alkylene, $C_{2-8}$ branched alkylene, $C_{2-4}$alkenylene, $C_{3-4}$alkynylene, $C_{3-6}$cycloalkylene, phenylene, hydroxy-substituted $C_{1-4}$alkylene or a direct bond; and salts thereof; which comprises reacting a compound of formula

A—CO—X—CO—B

where either A is $Z^1$ and B is $Z^2$ or $OC_{1-3}$alkyl, or A and B together form an —O— link, with a 4-hydroxy dimeric indole-dihydroindole, ROH, and optionally removing a $C_{1-3}$ alkyl or $Z^2$ group to give the free acid in which Z is OH, or which comprises activating a compound of formula R—O—CO—X—COOH to yield a compound in which Z is $Z^1$ and optionally reacting the product with ammonia, hydrazine or a $C_{1-3}$ alkanol to yield a compound in which Z is $NH_2$, $NHNH_2$ or $OC_{1-3}$ alkyl.

2. A process according to claim 1 in which Z is OH, $OC_{1-3}$ alkyl, $Z^1$ or $Z^2$.

3. A process according to claim 2 for producing a compound in which Z is OH or $OC_{1-3}$ alkyl.

4. A process according to claim 2 for producing a compound in which Z is Br, Cl, $N_3$, succinimidoxy, phthalimidoxy, benzotriazolyloxy, methansulfonyloxy, tosyloxy or benzenesulphonyloxy.

5. A process according to claim 2 for producing a compound in which Z is $CCl_3CH_2O$, $CBr_3CH_2O$, $CH_2ICH_2O$, benzyloxy, methylbenzyloxy, *t*-butyloxy, allyloxy methoxybenzyloxy, nitrobenzyloxy, phenacyloxy, nitrophenacyloxy, methoxyphenacyloxy, methylphenacyloxy, diphenylmethoxy, trityloxy, trimethylsilyloxy.

6. A process according to claims 2 to 5 for producing a compound in which X is $C_{1-4}$ alkylene.

**Patentansprüche für die Vertragsstaaten: BE CH DE FR GB IT LI LU NL SE**

1. Verbindung der Formel I:

$$R—O—CO—X—CO—Z \quad (I)$$

worin R die Formel II hat

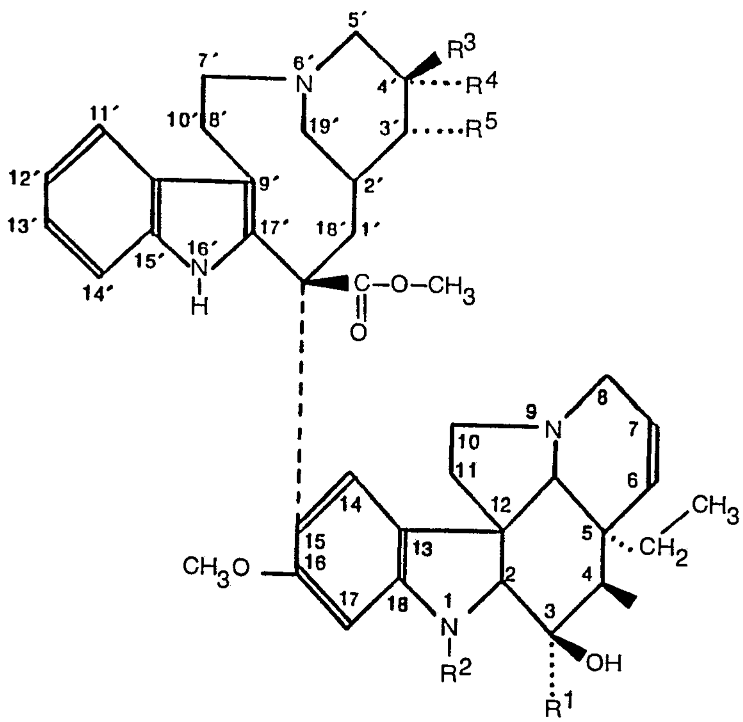

(II)

worin $R^2$ steht für H, $CH_3$ oder CHO; wenn $R^4$ und $R^5$ einzeln genommen werden, steht $R^5$ für H und einer der Reste $R^3$ und $R^4$ steht für Ethyl und der andere für H oder OH; wenn $R^4$ und $R^5$ zusammen mit den Kohlenstoffatomen, an die sie gebunden sind, genommen werden, bilden sie einen Oxiranring und $R^3$ steht für Ethyl; worin $R^1$ steht für COOH, $COOC_{1-3}$-Alkyl oder $COR^6$, worin $R^6$ steht für $NH_2$, NH—$C_{1-3}$-Alkyl, NH—$CH_2CH_2Cl$, 1-Pyrrolidyl, 1-Piperidinyl oder NH—$CH_2CH_2YCH_2$, worin Y für S oder O steht; worin Z für OH, $OC_{1-3}$-Alkyl, $NH_2$, $NHNH_2$, eine Carboxy-aktivierende Gruppe ($Z^1$) oder eine Carboxy-Schutzgruppe ($Z^2$) steht; und worin X steht für geradkettiges $C_{1-4}$-Alkylen, verzweigtes $C_{2-8}$-Alkylen, $C_{2-4}$-Alkenylen, $C_{3-4}$-Alkinylen, $C_{3-6}$-Cycloalkylen, Phenylen, Hydroxy-substituiertes $C_{1-4}$-Alkylen oder eine direkte Bindung; und ihre Salze.

2. Verbindung nach Anspruch 1, in der Z steht für OH, $OC_{1-3}$-Alkyl, $Z^1$ oder $Z^2$.

3. Verbindung nach Anspruch 2, in der Z steht für OH oder $OC_{1-3}$-Alkyl.

4. Verbindung nach Anspruch 2, in der Z steht für Br, Cl, $N_3$, Succinimidoxy, Phthalimidoxy, Benzotriazolyloxy, Methansulfonyloxy, Tosyloxy oder Benzosulfonyloxy.

5. Verbindung nach Anspruch 2, in der Z steht für $CCl_3CH_2O$, $CBr_3CH_2O$, $CH_2JCH_2O$, Benzyloxy, Me hylbenzyloxy, t-Butyloxy, Allyloxy, Methoxybenzyloxy, Nitrobenzyloxy, Phenacyloxy, Nitrophenacyloxy, Methoxyphenacyloxy, Methylphenacyloxy, Diphenylmethoxy, Trityloxy, Trimethylsilyloxy.

6. Verbindung nach einem der Ansprüche 2 bis 5, in der X steht für $C_{1-4}$-Alkylen.

7. Pharmazeutische Formlierung, die eine Verbindung, wie sie in Anspruch 1 definiert ist, in der Z steht für OH, $OC_{1-3}$-Alkyl, $NH_2$ oder $NHNH_2$, und ein pharmazeutisch akzeptables Verdünnungsmittel oder einen pharmazeutisch akzeptablen Träger enthält.

8. Verfahren zur Herstellung einer Verbindung, wie sie in Anpsruch 1 definiert ist, das umfaßt die Umsetzung einer Verbindung der Formel

$$A—CO—X—CO—B$$

worin entweder A steht für $Z^1$ und B steht für $Z^2$ oder $OC_{1-3}$-Alkyl oder A und B gemeinsam eine —O-Brücke bilden, mit einem dimeren 4-Hydroxy-indol-dihydroindol, ROH, und gegebenenfalls Entfernung einer $C_{1-3}$-Alkyl- oder $Z^2$-Gruppe unter Bildung der freien Säure, worin Z für OH steht, oder das umfaßt die Aktivierung einer Verbindung der Formel R—O—CO—X—COOH unter Bildung einer Verbindung, in der Z für $Z^1$ steht, und gegebenenfalls die Umsetzung des Produkts mit Ammoniak, Hydrazin oder einem $C_{1-3}$-Alkanol unter Bildung einer Verbindung, in der Z für $NH_2$, $NHNH_2$ oder $OC_{1-3}$-Alkyl steht.

**Patentansprüche für der Vertragsstaat: AT**

1. Verfahren zur Herstellung einer Verbindung der Formel I

$$R—O—CO—X—CO—Z \qquad (I)$$

worin R die Formel hat

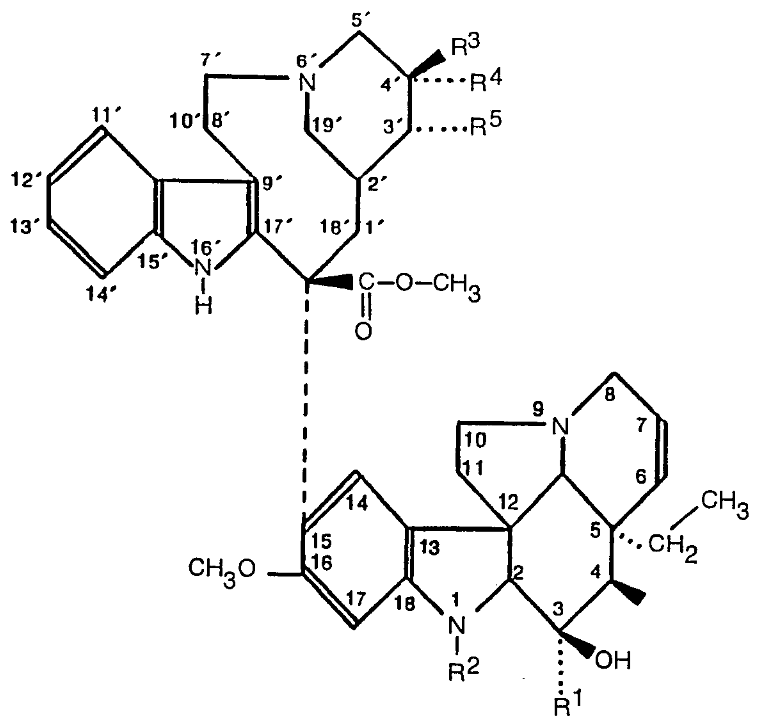

(II)

worin $R^2$ steht für H, $CH_3$ oder CHO; wenn $R^4$ und $R^5$ einzeln genommen werden, steht $R^5$ für H und einer der Reste $R^3$ und $R^4$ steht für Ethyl und der andere für H oder OH; wenn $R^4$ und $R^5$ zusammen mit den Kohlenstoffatomen, an die sie gebunden sind, genommen werden, bilden sie einen Oxiranring und $R^3$ steht für Ethyl; worin $R^1$ steht für COOH, $COOC_{1-3}$-Alkyl oder $COR^6$, worin $R^6$ steht für $NH_2$, NH—$C_{1-3}$-Alkyl, NH—$CH_2CH_2Cl$, 1-Pyrrolidyl, 1-Piperidinyl oder NH—$CH_2CH_2YCH_2$, worin Y für S oder O steht; worin Z für OH, $OC_{1-3}$-Alkyl, $NH_2$, $NHNH_2$, eine Carboxy-aktivierende Gruppe ($Z^1$) oder eine Carboxy-Schutzgruppe ($Z^2$) steht; und worin X steht für geradkettiges $C_{1-4}$-Alkylen, verzweigtes $C_{2-8}$-Alkylen, $C_{2-4}$-Alkenylen, $C_{3-4}$-Alkinylen, $C_{3-6}$-Cycloalkylen, Phenylen, Hydroxy-substituiertes $C_{1-4}$-Alkylen oder eine direkte Bindung; und ihre Salze das umfaßt die Umsetzung einer Verbindung der Formel

$$A—CO—X—CO—B$$

worin entweder A steht für $Z^1$ und B steht für $Z^2$ oder $OC_{1-3}$-Alkyl oder A und B gemeinsam eine —O-Brücke bilden, mit einem dimeren 4-Hydroxy-indol-dihydroindol, ROH, und gegebenenfalls Entfernung einer $C_{1-3}$-Alkyl- oder $Z^2$-Gruppe unter Bildung der freien Säure, in der Z für OH steht, oder das umfaßt die Aktivierung einer Verbindung der Formel R—O—CO—X—COOH unter Bildung einer Verbindung, in der Z für $Z^1$ steht, und gegebenenfalls die Umsetzung des Produkts mit Ammoniak, Hydrazin oder einem $C_{1-3}$-Alkanol unter Bildung einer Verbindung, in der Z für $NH_2$, $NHNH_2$ oder $OC_{1-3}$-Alkyl steht.

2. Verfahren nach Anspruch 1, worin Z für OH, $OC_{1-3}$-Alkyl, $Z^1$ oder $Z^2$ steht.

3. Verfahren nach Anspruch 2 zur Herstellung einer Verbindung, in der Z für OH oder $OC_{1-3}$-Alkyl steht.

4. Verfahren nach Anspruch 2 zur Herstellung einer Verbindung, in der Z für Br, Cl, $N_3$, Succinimidoxy, Phthalimidoxy, Benzotriazolyloxy, Methansulfonyloxy, Tosyloxy oder Benzosulfonyloxy steht.

5. Verfahren nach Anspruch 2 zur Herstellung einer Verbindung, in der Z für $CCl_3CH_2O$, $CBr_3CH_2O$, $CH_2JCH_2O$, Benzyloxy, Methylbenzyloxy, t-Butyloxy, Allyloxy, Methoxybenzyloxy, Nitrobenzyloxy, Phenacyloxy, Nitrophenacyloxy, Methoxyphenacyloxy, Methylphenacyloxy, Diphenylmethoxy, Trityloxy, Trimethylsilyloxy steht.

6. Verfahren nach einem der Ansprüche 2 bis 5 zur Herstellung einer Verbindung, in der X für $C_{1-4}$-Alkylen steht.

**Revendications pour les Etats contractants: BE CH DE FR GB IT LI LU NL SE**

1. Composé de formule I

R—O—CO—X—CO—Z (I)

où R répond à la formule II

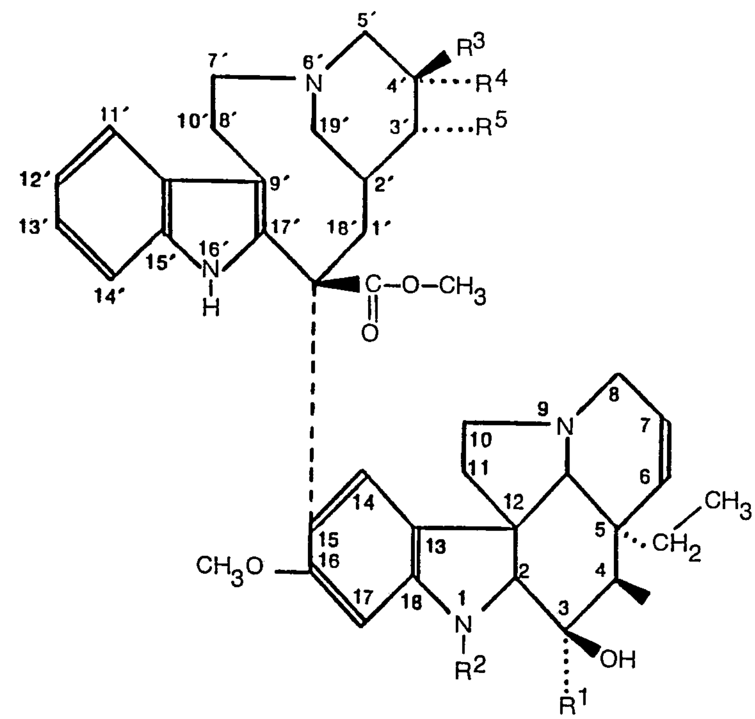

(II)

dans laquelle $R^2$ représente H, $CH_3$ ou CHO; lorsque $R^4$ et $R^5$ sont pris séparément, $R^5$ représente H et un des radicaux $R^3$ et $R^4$ représente un groupe éthyle, l'autre représentant H ou OH; lorsque $R^4$ et $R^5$ sont pris ensemble avec les atomes de carbone auxquels ils sont fixés, ils constituent un noyau oxiranne, et $R^3$ représente un groupe éthyle; dans laquelle $R^1$ représente COOH, COO-alkyle en $C_{1-3}$ ou CO—$R^6$, où $R^6$ représente $NH_2$, NH-alkyle en $C_{1-3}$, NH—$CH_2CH_2Cl$, un groupe 1-pyrrolidyl, un groupe 1-pipéridinyl, NH-$CH_2CH_2YCH_3$, où Y représente S ou O; dans laquelle Z représente OH, O-alkyle en $C_{1-3}$, $NH_2$, $NHNH_2$ ou un groupe activateur du groupe carboxyle ($Z^1$) ou encore un groupe protecteur du groupe carboxyle ($Z^2$); et dans laquelle X représente un groupe alkylène à chaîne droite en $C_{1-4}$, un groupe alkylène à chaîne ramifiée en $C_{2-8}$, un groupe alcénylène en $C_{2-4}$, un groupe alcynylène en $C_{3-4}$, un groupe cycloalkylène en $C_{3-6}$, un groupe phénylène, un groupe alkylène en $C_{1-4}$ substitué par un groupe hydroxyle ou encore une liaison directe; ainsi que ses sels.

2. Composé selon la formule I dans laquelle Z représente OH, O-alkyl en $C_{1-3}$, $Z^1$ ou $Z^2$.

3. Composé selon la revendication 2 dans laquelle Z représente OH ou un groupe O-alkyl en $C_{1-3}$.

4. Composé selon la revendication 2 dans lequel Z représente Br, Cl, $N_3$, un groupe succinimidoxy, un groupe phtalimidoxy, un groupe benzotriazolyloxy, un groupe méthanesulfonyloxy, un groupe tosyloxy ou un groupe benzènesulfonyloxy.

5. Composé selon la revendication 2 dans lequel Z représente $CCl_3CH_2O$—, $CBr_3CH_2O$—, $CH_2ICH_2O$—, un groupe benzyloxy, un groupe méthylbenzyloxy, un groupe t-butyloxy, un groupe allyloxy, un groupe méthoxybenzyloxy, un groupe nitrobenzyloxy, un groupe phénacyloxy, un groupe nitrophénacyloxy, un groupe méthoxyphénacyloxy, un groupe méthylphénacyloxy, un groupe diphénylméthoxy, un groupe trityloxy, un groupe triméthylsilyloxy.

6. Composé selon l'une quelconque des revendications 2 à 5, dans lequel X représente un groupe alkylène en $C_{1-4}$.

7. Formulation pharmaceutique comprenant un composé tel que défini dans la revendication 1, dans lequel Z représente OH, O-alkyl en $C_{1-3}$, $NH_2$ ou $NHNH_2$, ainsi qu'un diluant ou un support pharmaceutiquement acceptable.

8. Procédé de préparation d'un composé tel que défini dans la revendication 1, consistant à faire réagir un composé de formule

A—CO—X—CO—B

où A représente $Z^1$ et B représente $Z^2$ ou un groupe O-alkyl en $C_{1-3}$, ou bien A et B, pris ensemble, forment une liaison —O—, avec un indole-dihydroindole dimère 4-hydroxy, à savoir ROH, et éventuellement à éliminer un groupe alkyle en $C_{1-3}$ ou un groupe $Z^2$, pour obtenir l'acide libre dans lequel Z représente OH, ou encore consistant à activer un composé de formule R—O—CO—X— COOH pour obtenir un composé dans lequel Z représente $Z^1$ et éventuellement faire réagir le composé avec de l'ammoniac, de l'hydrazine ou un alcanol en $C_{1-3}$, pour obtenir un composé dans lequel Z représente $NH_2$, $NHNH_2$ ou un groupe O-alkyl en $C_{1-3}$.

**Revendications pour l'Etat contractant: AT**

1. Procédé de préparation d'un composé de formule I

R—O—CO—X—CO—Z (I)

où R répond à la formule

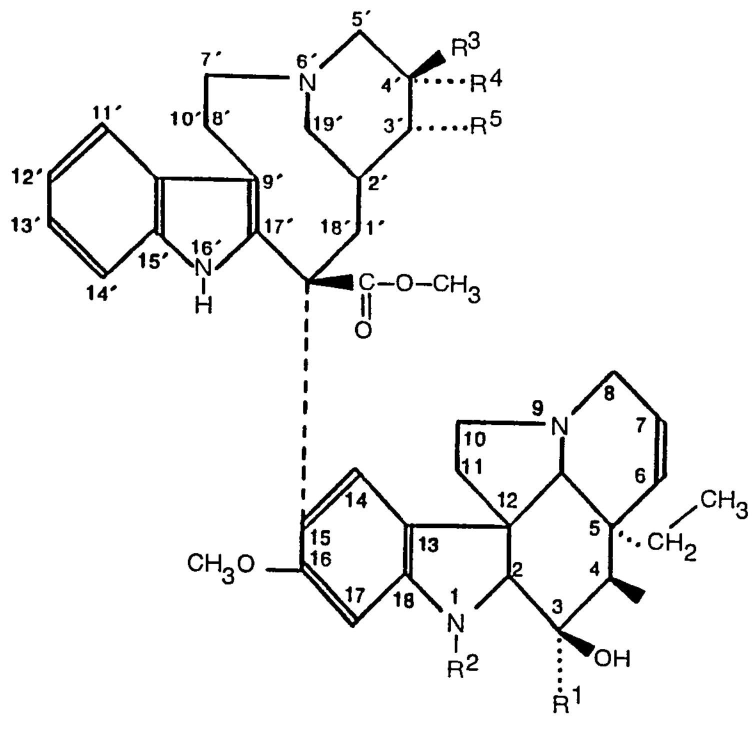

(II)

dans laquelle $R^2$ représente H, $CH_3$ ou CHO; lorsque $R^4$ et $R^5$ sont pris séparément, $R^5$ représente H et un des radicaux $R^3$ et $R^4$ représente un groupe éthyle, l'autre représentant H ou OH; lorsque $R^4$ et $R^5$ sont pris ensemble avec les atomes de carbone auxquels ils sont fixés, ils constituent un noyau oxiranne, et $R^3$ représente un groupe éthyle; dans laquelle $R^1$ représente COOH, COO-alkyle en $C_{1-3}$ ou CO—$R^6$, où $R^6$ représente $NH_2$, NH-alkyle en $C_{1-3}$, NH—$CH_2CH_2Cl$, un groupe 1-pyrrolidyle, un groupe 1-pipéridinyle, NH-$CH_2CH_2YCH_3$, où Y représente S ou O; dans laquelle Z représente OH, O-alkyle en $C_{1-3}$, $NH_2$, $NHNH_2$ ou un groupe activateur du groupe carboxyle ($Z^1$) ou encore un groupe protecteur du groupe carboxyle ($Z^2$); et dans laquelle X représente un groupe alkylène à chaîne droite en $C_{1-4}$, un groupe alkylène à chaîne ramifiée en $C_{2-8}$, un groupe alcénylène en $C_{2-4}$, un groupe alcynylène en $C_{3-4}$, un groupe cycloalkylène en $C_{3-6}$, un groupe phénylène, un groupe alkylène en $C_{1-4}$ substitué par un groupe hydroxyle ou encore une liaison directe; ainsi que ses sels; ce procédé consistant à faire réagir un composé de formule

A—CO—X—CO—B

où A représente $Z^1$ et B représente $Z^2$ ou un groupe O-alkyl en $C_{1-3}$, ou bien A et B, pris ensemble, forment une liaison —O—, avec un indole-dihydroindole dimère 4-hydroxy, à savoir ROH, et éventuellement à

éliminer un groupe alkyle en $C_{1-3}$ ou un groupe $Z^2$, pour obtenir l'acide libre dans lequel Z représente OH, ou encore consistant à activer un composé de formule R—O—CO—X— COOH pour obtenir un composé dans lequel Z représente $Z^1$ et éventuellement faire réagir le compose avec de l'ammoniac, de l'hydrazine ou un alcanol en $C_{1-3}$, pour obtenir un composé dans lequel Z représente $NH_2$, $NHNH_2$ ou un groupe O-alkyl en $C_{1-3}$.

2. Procédé selon la revendication 1, dans laquelle Z représente OH, O-alkyl en $C_{1-3}$, $Z^1$ ou $Z^2$.

3. Procédé selon la revendication 2, dans laquel Z représenté OH ou un groupe O-alkyl en $C_{1-3}$.

4. Procédé selon la revendication 2 pour préparer un composé dans lequel Z représente Br, Cl, $N_3$, un groupe succinimidoxy, un groupe phtalimidoxy, un groupe benzotriazolyloxy, un groupe méthanesulfonyloxy, un groupe tosyloxy ou un groupe benzènesulfonyloxy.

5. Procédé selon la revendication 2 pour la préparation d'un composé dans lequel Z représente $CCl_3CH_2O$—, $CBr_3CH_2O$—, $CH_2ICH_2O$—, un groupe benzyloxy, un groupe méthylbenzyloxy, un groupe t-butyloxy, un groupe allyloxy, un groupe méthoxybenzyloxy, un groupe nitrobenzyloxy, un groupe phénacyloxy, un groupe nitrophénacyloxy, un groupe méthoxyphénacyloxy, un groupe méthylphénacyloxy, un groupe diphénylméthoxy, un groupe trityloxy, un groupe triméthylsilyloxy.

6. Procédé selon l'une quelconque des revendications 2 à 5, pour préparer un composé dans lequel X représente un groupe alkylène en $C_{1-4}$.